# EUROPEAN PATENT SPECIFICATION

(11) **EP 0 983 068 B1**
(45) Date of publication and mention of the grant of the patent: **28.11.2001**
(21) Application number: 98924313.4
(22) Date of filing: 14.05.1998
(51) Int. Cl.: A61K 31/495

(54) **THE USE OF 1,2,4-TRIAZOLO 1,5-c]PYRIMIDINE HETEROCYCLIC ANALOGUES FOR THE PREPARATION OF MEDICAMENTS USEFUL FOR THE TREATMENT OF CEREBROVASCULAR DISTURBANCES**
VERWENDUNG VON 1,2,4-TRIAZOLO(1,5-C)PYRIMIDINE HETEROCYCLISCHEN ANALOGEN ZUR HERSTELLUNG VON ARZNEIMITTELN ZUR BEHANDLUNG VON ZEREBROVASCULAREN STÖRUNGEN
UTILISATION D'ANALOGUES HETEROCYCLIQUES DE 1,2,4-TRIAZOLO 1,5-C]PYRIMIDINE POUR LA PREPARATION DE MEDICAMENTS S'UTILISANT DANS LE TRAITEMENT D'ACCIDENTS VASCULAIRES CEREBRAUX

(30) Priority: 21.05.1997 IT MI971190
(43) Date of publication of application: 08.03.2000
(73) Proprietor: SCHERING-PLOUGH S.p.A., 20141 Milano (IT)
(72) Inventor: ONGINI, Ennio, I-20090 Segrate (IT); ADAMI, Marina, I-20097 S. Donato Milanese (IT); BERTORELLI, Rosalia, I-20125 Milano (IT)
(74) Representative: Minoja, Fabrizio, Dr.
(86) International application number: EP9802852
(87) International publication number: WO9852568

(56) References cited:
- ONGINI: "a selective a(2a) adenosine receptor antagonist" DRUG DEV. RES., vol. 42, no. 2, October 1997, pages 63-50, XP002076516
- BONA ET AL.: "neonatal cerebral hypoxia-ischemia: the effect of adenosine receptor antagonists" NEUROPHARMACOLOGY, vol. 36, no. 9, September 1997, pages 1327-1338, XP002076517
- BARALDI ET AL: "pyrazolo(4,3-3)-1,2,4-triazolo(1,5-c)pyri midine derivatives: potent and selective a2a adenosine antagonists" J MED CHEM, vol. 39, no. 5, 1996, pages 1164-1171, XP002076518
- DIONISOTTI ET AL.: "effects of the new a2 adenosine receptor antagonist 8fb-ptp an 8 substituted pyrazolo-triazolo-pyrimidine on in vitro functional models" BR J PHARMACOL, vol. 112, no. 2, 1994, pages 659-665, XP002076519

## Description

The present invention relates to the use of 1,2,4-triazolo[1,5-c]pyrimidine heterocyclic analogues of formula (I) 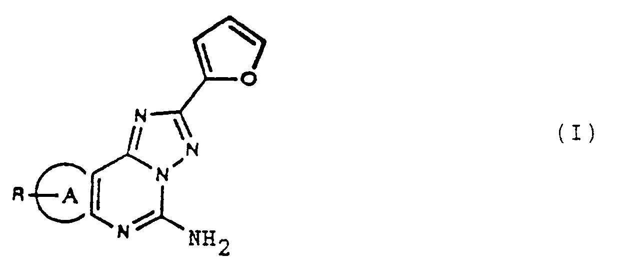 in which:
A is a pyrazole, imidazole or triazole ring;
R is hydrogen; C₁-C₈ alkyl; C₃-C₇ alkenyl, C₃-C₇ alkynyl; C₃-C₇ cycloalkyl; C₁-C₅ alkyl substituted with 1-3 halogen atoms, hydroxy, C₁-C₄ alkoxy, C₃-C₇ cycloalkyl, groups of formula -NR₁R₂, -CONR₁R₂, wherein R₁ and R₂, which can be the same or different, are hydrogen, C₁-C₅ alkyl, C₇-C₁₀ aralkyl, phenyl, or taken together with the nitrogen atom they are linked to, they form an azetidine ring or a 5-6 membered heterocyclic ring containing one or more heteroatoms selected from N, O, S; aryl optionally substituted with halogen atoms, C₁-C₄ alkoxy, C₁-C₄ alkyl, nitro, amino, cyano, C₁-C₄ haloalkyl, C₁-C₄ haloalkoxy, carboxy, carboxyamido groups; C₇-C₁₀ aralkyl in which the aryl moiety can be substituted with one or more of the substituents indicated above for the aryl group; a group of formula 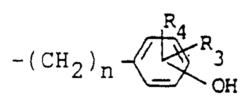 wherein R ₃ and R ₄ which can be the same or different, are H, OH, halogen atoms, C₁-C₄ alkoxy, C₁-C₄ alkyl, nitro, amino, cyano, C₁-C₄ haloalkyl, C₁-C₄ haloalkoxy, carboxy or carboxyamido groups; moreover the OH group, together with one of R₃ or R₄, or R₃ and R₄ together, can form the methylenedioxy group -O-CH₂-O-, n is an integer of 0 to 4; a group of formula -(CH₂)ₘ-Het, wherein Het is a 5-6 membered aromatic or non aromatic heterocyclic ring containing one or more heteroatoms selected from N, O, S and m is an integer of 1 to 5;
or a pharmaceutically acceptable salt thereof, for the preparation of a medicament for the treatment of cerebrovascular disorders, i.e. in all those brain injuries caused by either impairments of the cerebral circulation or trauma, following deprivation of oxygen and of those nutritional substances which the area vascularized by the vessels involved in the pathological condition is subjected to. Stroke, cerebral infarction and brain trauma are among the most severe conditions which can be treated with the medicaments here described.

The compounds of formula (I) are selective antagonists of adenosine A_{2A} receptors.

Adenosine is known to be an endogenous modulator of a number of physiological functions. At the cardiovascular system level, adenosine is a strong vasodilator and a cardiac depressor. On central nervous system, adenosine induces sedative, anxiolytic and antiepileptic effects. On the respiratory system, adenosine induces bronchoconstriction. At the kidney level, it exerts a biphasic action, inducing vasoconstriction at low concentrations and vasodilation at high doses. Adenosine acts as a lipolysis inhibitor on fat cells and as an antiaggregant on platelets (Stone T.W., Purine receptors and their pharmacological roles. In: Advances in drug research. Academic Press Limited, 1989, 18, 291-429; Progress Cardiovasc. Dis. 1989, 32, 73-97; Williams M., Adenosine and Adenosine receptors. The Humana Press, 1990).

A number of studies showed adenosine actions are mediated by four subtypes of receptors which are located on the cell membrane: two high-affinity ones, inhibiting the activity of the enzyme adenylate cyclase (A₁ and A₃ receptors), and two low-affinity ones, stimulating the activity of the same enzyme (A_{2A} and A_{2B} receptors) (J. Med. Chem. 1982, 25, 197-207; Physiol. Rev. 1990, 70, 761-845; J. Med. Chem. 1992, 35, 407-422; Pharmacol. Rev. 1994, 46, 143-156).

Intense research efforts have made it possible to identify and develop analogs of adenosine which are able to interact as selective agonists for the four receptors, including the A_{2A} receptor type (Pharmacol. Rev., 1994, 46, 143-156).

Other studies allowed to develop heterocyclic compounds capable of antagonizing some receptor types. The xanthine compounds, for example, antagonize both A₁ and A_{2A} receptors (J. Med. Chem., 1992, 35, 407-422).

As far as the A_{2A} receptor antagonists are concerned, the compounds of general formula (I), which are known to exert a selective action on said receptors, as well as the process for the preparation thereof, are disclosed in WO 9501356 and WO 9705138 applications. A number of different possible uses of the compounds of formula (I) are cited in said applications, but in no cases a specific use in the treatment of cerebrovascular disorders is described.

Now it has surprisingly been found that compounds of general formula (I) are capable of reducing by more than 40% the total volume of cerebral infarction in animal models in which a focal cerebral ischemia has been induced.

Particularly, the study was carried out on animals (rats) subjected to occlusion of the median cerebral artery (MCA), by electrocauterization and subsequent determination of the cerebral infarction total volume by means of histologic analysis of the brain preparations (Surg. Neurol. 1985, 24:47-51).

Said models are considered relevant to cerebrovascular pathologies in humans.

Although other heterocyclic compounds (CGS 15943 and CP66713, respectively; Life Sciences, 55, 61-65, 1994 and Brain Research 705, 79-84, 1995) are known to act favourably in cerebral ischemia animal models, nevertheless such compounds act as non-selective antagonists of the A_{2A} receptors, in that they also block other adenosine receptor subtypes thus causing undesired side-effects.

On the contrary, the compounds of formula (I) showed a high affinity for A_{2A} receptors and a remarkable selectivity compared with the other receptors subtypes, having, for instance, a A_{2A} receptor affinity up to 800-fold higher than the affinity to A₁ receptors, therefore being safer and more suitable even for a long-term treatment of disturbances due to cerebrovascular pathologies.

Particularly effective and therefore preferred are those compounds of formula (I) wherein:
A is pyrazole, imidazole or triazole;
R is C₇-C₁₀ aralkyl or the group 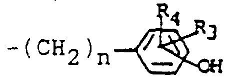 wherein R₃ and R₄, which can be the same or different, are hydrogen, OH, halogen, C₁-C₄ alkoxy, C₁-C₄ alkyl, nitro, amino, cyano, C₁-C₄ haloalkoxy, C₁-C₄ haloalkyl, carboxy or carboxyamido; moreover the OH group, together with one of R₃ or R₄, or R₃ and R₄ together, can form the methylenedioxy group -O-CH₂-O-; n is an integer of 0 to 4,
most preferred are the compounds having the following formulae (II-IV): 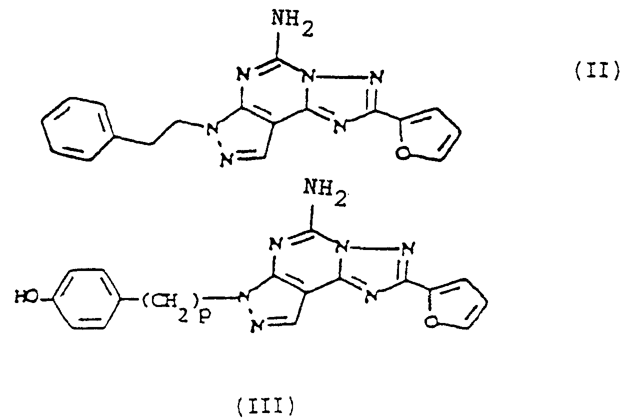 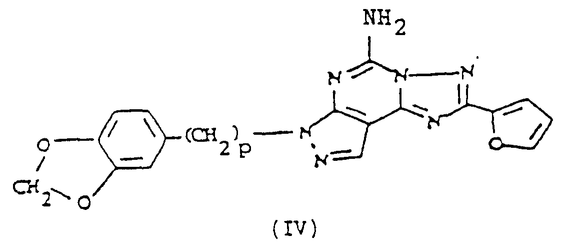 wherein p = 2 or 3.

For the envisaged therapeutical uses, compounds I will be formulated as suitable pharmaceutical compositions, which can be administered, for example, by the oral, parenteral or transdermal routes, using known techniques and excipients, as described for example in Remington's Pharmaceutical Sciences Handbook, Mack Pub. Co., NY, USA, 17th ed., 1985.

The daily dosage will depend, of course, on many factors (severity of the pathology to treat, patient conditions, toxicology and pharmacokinetic of the selected compound) but generally it will range from 0.01 to 1 mg/kg body weight.

Examples of pharmaceutical compositions comprise capsules, tablets, solutions, syrups, vials, controlled-release forms, transdermal forms (plasters) and the like.

## Claims

1. The use of the compounds of formula I : 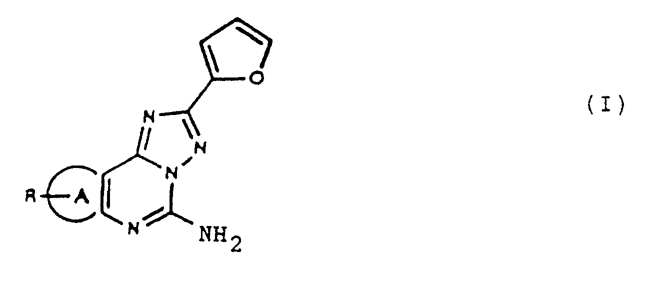 in which:
A is a pyrazole, imidazole or triazole ring;
R is hydrogen; C₁-C₈ alkyl; C₃-C₇ alkenyl, C₃-C₇ alkynyl; C₃-C₇ cycloalkyl; C₁-C₅ alkyl substituted with 1-3 halogen atoms, hydroxy, C₁-C₄ alkoxy, C₃-C₇ cycloalkyl, groups of formula -NR₁R₂, -CONR₁R₂, wherein R₁ and R₂, which can be the same or different, are hydrogen, C₁-C₅ alkyl, C₇-C₁₀ aralkyl, phenyl, or taken together with the nitrogen atom they are linked to, they form an azetidine ring or a 5-6 membered heterocyclic ring containing one or more heteroatoms selected from N, O, S; aryl optionally substituted with halogen atoms, C₁-C₄ alkoxy, C₁-C₄ alkyl, nitro, amino, cyano, C₁-C₄ haloalkyl, C₁-C₄ haloalkoxy, carboxy, carboxyamido groups; C₇-C₁₀ aralkyl in which the aryl moiety can be substituted with one or more of the substituents indicated above for the aryl group; a group of formula 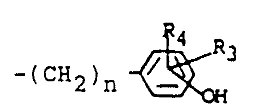 wherein R₃ e R₄ which can be the same or different, are H, OH, halogen atoms, C₁-C₄ alkoxy, C₁-C₄ alkyl, nitro, amino, cyano, C₁-C₄ haloalkyl, C₁-C₄ haloalkoxy, carboxy or carboxyamido groups; moreover the OH group, together with one of R₃ or R₄, or R₃ and R₄ together, can form the methylenedioxy group -O-CH₂-O-, n is an integer of 0 to 4; a group of formula -(CH₂)ₘ-Het, wherein Het is a 5-6 membered aromatic or non aromatic heterocyclic ring containing one or more heteroatoms selected from N, O, S and m is an integer of 1 to 5;
or a pharmaceutically acceptable salt thereof, for the preparation of a medicament for the treatment of cerebrovascular disorders, such as stroke, cerebral infarction and brain trauma.

2. The use according to claim 1 of the compounds in which:
A is pyrazole, imidazole or triazole;
R is C₇-C₁₀ aralkyl or the group 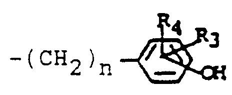 wherein R₃ and R₄, which can be the same or different, are hydrogen, OH, halogen, C₁-C₄ alkoxy, C₁-C₄ alkyl, nitro, amino, cyano, C₁-C₄ haloalkoxy, C₁-C₄ haloalkyl, carboxy or carboxyamido; moreover the OH group, together with one of R₃ or R₄, or R₃ and R₄ together, can form the methylenedioxy group -O-CH₂-O-; n is an integer of 0 to 4.

3. The use according to claim 2 of the compound of formula (II)

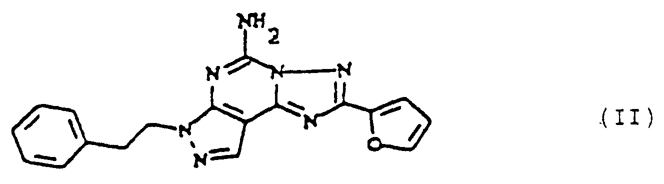

4. The use according to claim 2 of the compounds of formula (III) 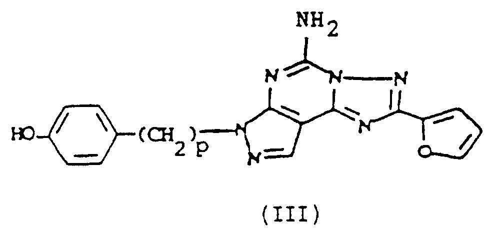 wherein p = 2 or 3.

5. The use according to claim 2 of the compounds of formula (IV) 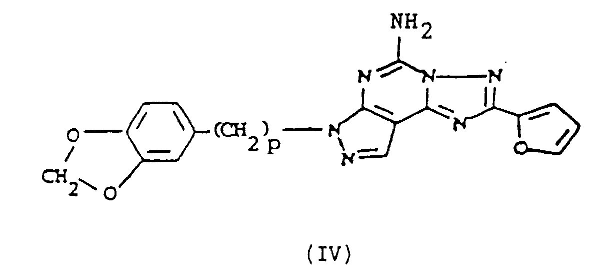 wherein p = 2 or 3.

## Patentansprüche

1. Verwendung von Verbindungen der Formel I: 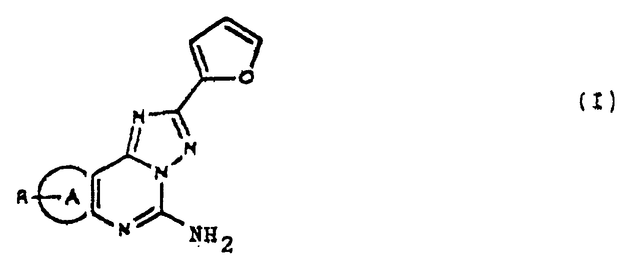 worin
A ein Pyrazol-, Imidazol- oder Triazolring bedeutet;
R Wasserstoff, C₁-C₈-Alkyl, C₃-C₇-Alkenyl, C₃-C₇-Alkinyl; C₃-C₇-Cyclo-alkyl; C₁-C₅-Alkyl, substituiert mit 1 bis 3 Halogenatomen, Hydroxy, C₁-C₄-Alkoxy, C₃-C₇-Cycloalkyl, Gruppen der Formel -NR₁R₂, -CONR₁R₂, worin R₁ und R₂, die gleich oder unterschiedlich sein können, Wasserstoff, C₁-C₅-Alkyl, C₇-C₁₀-Aralkyl, Phenyl bedeuten oder zusammen mit dem Stickstoffatom, an das sie gebunden sind, einen Azetidinring oder einen 5- bis 6-gliedrigen heterocyclischen Ring, enthaltend ein oder mehrere Heteroatome, ausgewählt aus N, O, S, bedeuten; Aryl, gegebenenfalls substituiert mit Halogenatomen, C₁-C₄-Alkoxy-, C₁-C₄-Alkyl-, Nitro-, Amino-, Cyano-, C₁-C₄-Halogenalkyl-, C₁-C₄-Halogenalkoxy-, Carboxy-, Carboxyamido-Gruppen; C₇-C₁₀-Aralkyl, worin die Arylgruppierung mit einem oder mehreren Substituenten, wie oben für die Arylgruppe angegeben, substituiert sein kann; eine Gruppe der Formel 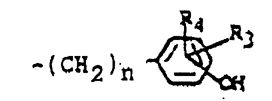 worin R₃ und R₄, die gleich oder unterschiedlich sein können, H, OH, Halogenatome, C₁-C₄-Alkoxy-, C₁-C₄-Alkyl-, Nitro-, Amino-, Cyano-, C₁-C₄-Halogen-alkyl-, C₁-C₄-Halogenalkoxy-, Carboxy- oder Carboxyamidogruppen bedeuten; weiterhin die OH-Gruppe zusammen mit einem von R₃ oder R₄ oder R₃ und R₄ zusammen die Methylendioxygruppe -O-CH₂-O-bilden kann, n eine ganze Zahl von 0 bis 4 bedeutet; eine Gruppe der Formel -(CH₂)ₘ-Het, worin Het ein 5- bis 6-gliedriger aromatischer oder nicht-aromatischer heterocyclischer Ring ist, der ein oder mehrere Heteroatome, ausgewählt aus N, O, S, enthält und m eine ganze Zahl von 1 bis 5 bedeutet, bedeutet;
oder eines pharmazeutisch annehmbaren Salzes davon für die Herstellung eines Arzneimittels zur Behandlung von zerebrovaskulären Störungen, wie Schlaganfall, Hirninfarkt und Gehirntrauma.

2. Verwendung nach Anspruch 1 von Verbindungen, worin
A Pyrazol, Imidazol oder Triazol bedeutet;
R C₇-C₁₀-Aralkyl oder die Gruppe 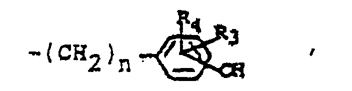 bedeutet, worin
R₃ und R₄, die gleich oder unterschiedlich sein können, Wasserstoff, OH, Halogen, C₁-C₄-Alkoxy, C₁-C₄-Alkyl, Nitro, Amino, Cyano, C₁-C4-Halogenalkoxy, C1-C4-Halogenalkyl, Carboxy oder Carboxyamido bedeuten; weiterhin die OH-Gruppe zusammen mit einem von R3 oder R4 oder R3 und R4 zusammen die Methylendioxygruppe -O-CH₂-O- bilden kann; n eine ganze Zahl von 0 bis 4 bedeutet.

3. Verwendung nach Anspruch 2 der Verbindung der Formel (II)

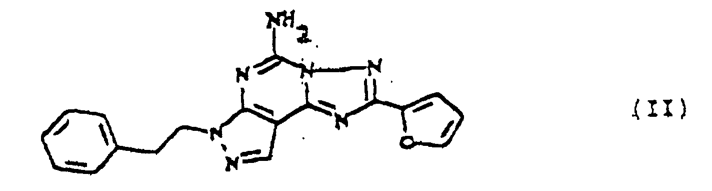

4. Verwendung nach Anspruch 2 der Verbindung der Formel (III) 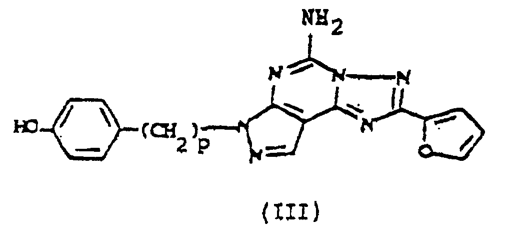 worin p = 2 oder 3.

5. Verwendung nach Anspruch 2 der Verbindungen der Formel (IV) 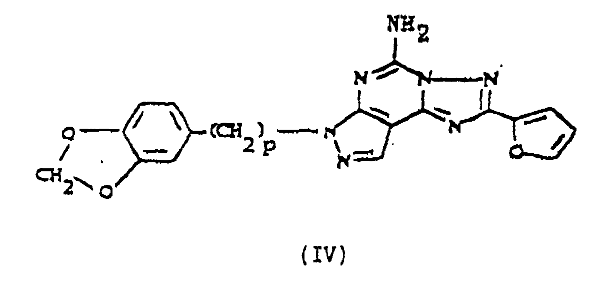 worin p = 2 oder 3.

## Revendications

1. Utilisation d'un composé de formule I : 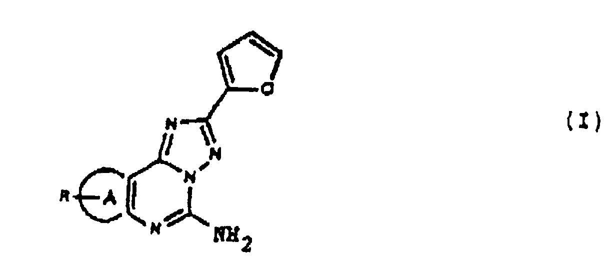 dans laquelle :
A est un noyau pyrazole, imidazole ou triazole ;
R est de l'hydrogène ; un groupe alkyle en C₁ à C₈ ; alcényle en C₃ à C₇ ; alcynyle en C₃ à C₇ ; cycloalkyle en C₃ à C₇ ; alkyle en C₁ à C₅ substitué par 1 à 3 atomes d'halogène, hydroxy, alcoxy en C₁ à C₄, cycloalkyle en C₃ à C₇, des groupes de formule -NR₁R₂, -CONR₁R₂ où R₁ et R₂, qui peuvent être identiques ou différents, sont de l'hydrogène, un groupe alkyle en C₁ à C₅, aralkyle en C₇ à C₁₀, phényle ou, pris ensemble avec l'atome d'azote auquel ils sont liés, ils forment un noyau azétidine ou un noyau hétérocyclique de 5 à 6 chaînons contenant un ou plusieurs hétéroatomes choisis parmi N, O, S ; des groupes aryle éventuellement substitué par des atomes d'halogène, alcoxy en C₁ à C₄, alkyle en C₁ à C₄, nitro, amino, cyano, halogénoalkyle en C₁ à C₄, halogénoalcoxy en C₁ à C₄, carboxy, carboxyamido ; aralkyle en C₇ à C₁₀ dans lequel la fraction aryle peut être substituée par un ou plusieurs des substituants indiqués ci-dessus pour le groupe aryle ; un groupe de formule 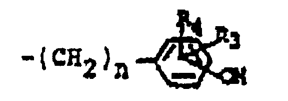 dans laquelle R₃ et R₄, qui peuvent être identiques ou différents, sont H, OH, des atomes d'halogène, des groupes alcoxy en C₁ à C₄, alkyle en C₁ à C₄, nitro, amino, cyano, halogénoalkyle en C₁ à C₄, halogénoalcoxy en C₁ à C₄, carboxy ou carboxyamido ; en outre, le groupe OH, avec l'un parmi R₃ ou R₄, ou R₃ et R₄ ensemble, peuvent former le groupe méthylènedioxy -O-CH₂-O-, n est un entier de 0 à 4 ; un groupe de formule -(CH₂)ₘ-Het, où Het est un noyau aromatique ou non aromatique hétérocyclique à 5-6 chaînons contenant un ou plusieurs hétéroatomes choisis parmi N, O, S, et m est un entier de 1 à 5 ;
ou d'un sel pharmaceutiquement acceptable de celui-ci, pour la préparation d'un médicament pour le traitement de troubles cérébro-vasculaires, tels qu'un ictus, un infarctus cérébral et un traumatisme cérébral.

2. Utilisation selon la revendication 1 des composés dans lesquels :
A est un noyau pyrazole, imidazole ou triazole ;
R est un groupe aralkyle en C₇ à C₁₀ ou le groupe 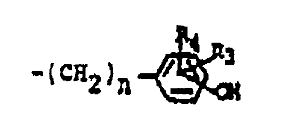
dans lequel R₃ et R₄, qui peuvent être identiques ou différents, sont de l'hydrogène, OH, un halogène, un groupe alcoxy en C₁ à C₄, alkyle en C₁ à C₄, nitro, amino, cyano, halogénoalcoxy en C₁ à C₄, halogénoalkyle en C₁ à C₄, carboxy ou carboxyamido ; en outre, le groupe OH, avec l'un parmi R₃ ou R₄, ou R₃ et R₄ ensemble, peuvent former le groupe méthylènedioxy -O-CH₂-O- ; n est un entier de 0 à 4.

3. Utilisation selon la revendication 2 du composé de formule (II)

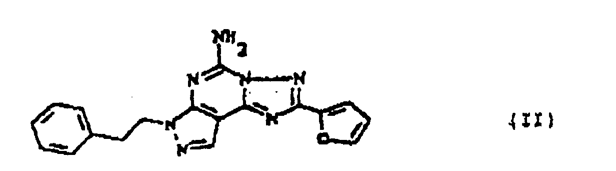

4. Utilisation selon la revendication 2 des composés de formule (III) 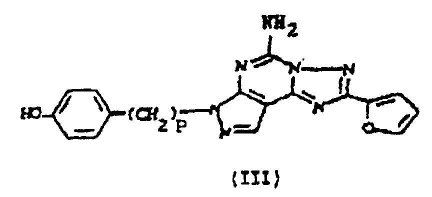 dans laquelle p = 2 ou 3.

5. Utilisation selon la revendication 2 des composés de formule (IV) 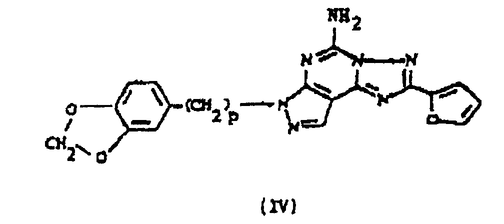 dans laquelle p = 2 ou 3.
